# EUROPEAN PATENT SPECIFICATION

(11) **EP 2 448 896 B1**
(45) Date of publication and mention of the grant of the patent: **12.03.2014**
(21) Application number: 10735189.2
(22) Date of filing: 28.06.2010
(51) Int. Cl.: C07C 29/40, C07C 35/08, C07C 209/62, C07C 211/35

(54) **METHOD OF PREPARING 1-HYDROXY-1,3,3,5,5-PENTAMETHYLCYCLOHEXANE**
VERFAHREN ZUR HERSTELLUNG VON 1-HYDROXY-1,3,3,5,5-PENTAMETHYLCYCLOHEXAN
PROCÉDÉ DE PRÉPARATION DE 1-HYDROXY -1,3,3,5,5-PENTAMETHYLCYCLOHEXANE

(30) Priority: 29.06.2009 EP 09008463; 29.06.2009 US 269772 P
(43) Date of publication of application: 09.05.2012
(73) Proprietor: Merz Pharma GmbH & Co. KGaA, 60318 Frankfurt (DE)
(72) Inventor: KOLLER, Herbert, A-Wien (AT); PYERIN, Michael, A-2345 Brunn am Gebirge (AT); SBROGIÓ, Federico, I-36075 Montecchio Maggiore (IT)
(74) Representative: Ricker, Mathias
(86) International application number: PCT/EP2010/003919
(87) International publication number: WO 2011/000536

(56) References cited:
- WO-A-99/01416
- US-A- 4 126 140
- DANYSZ W ET AL: "AMINO-ALKYL-CYCLOHEXANS AS A NOVEL CLASS OF UNCOMPETITIVE NMDA RECEPTOR ANTAGONISTS" CURRENT PHARMACEUTICAL DESIGN, BENTHAM SCIENCE PUBLISHERS, NL, vol. 8, no. 10, 1 January 2002 (2002-01-01), pages 835-843, XP008030349 ISSN: 1381-6128 cited in the application
- JIRGENSONS A ET AL: "Synthesis and structure-affinity relationships of 1,3,5-alkylsubstituted cyclohexylamines binding at NMDA receptor PCP site" EUROPEAN JOURNAL OF MEDICINAL CHEMISTRY, EDITIONS SCIENTIFIQUE ELSEVIER, PARIS, FR, vol. 35, no. 6, 1 June 2000 (2000-06-01), pages 555-565, XP004330448 ISSN: 0223-5234 cited in the application
- CHIURDOGLU G ET AL: "Alicyclic compounds with a quaternary carbon. I. Synthesis of some gem-polymethylcyclohexenes" BULLETIN DES SOCIETES CHIMIQUES BELGES, XX, XX, vol. 63, 1 January 1954 (1954-01-01), pages 357-378, XP009124894 ISSN: 0037-9646

## Description

### FIELD OF THE INVENTION

The present invention relates to a method of preparing 1-hydroxy-1,3,3,5,5-pentamethylcyclohexane. Said product may be used as an intermediate in the manufacture of 1-amino-1,3,3,5,5-pentamethylcyclohexane (Neramexane) or a pharmaceutically acceptable salt thereof.

### BACKGROUND OF THE INVENTION

1-amino-1,3,3,5,5-pentamethylcyclohexane (Neramexane) and pharmaceutically acceptable salts thereof are valuable agents for the continuous therapy of patients suffering from diseases and conditions such as tinnitus, and nystagmus.

Methods of preparing these agents are already known.

In one method, commercially available isophorone is converted to Neramexane in a reaction sequence comprising five steps according to the following reaction scheme (W. Danysz et al., Current Pharmaceutical Design, 2002, 8, 835-843):

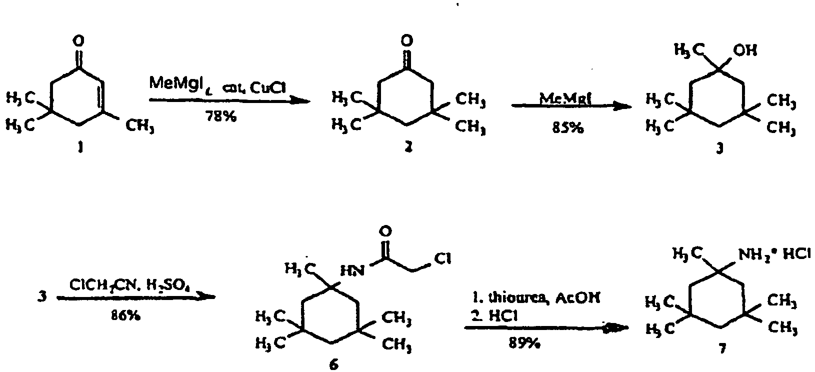

In the first step of the sequence, isophorone **1** is converted into 3,3,5,5-tetramethylcyclohexanone 2 by CuCl-catalyzed conjugate addition of methylmagnesium iodide. The yield of target compound is 78 % by weight.

In the second step, 3,3,5,5-tetramethylcyclohexanone **2** is converted into 1,3,3,5,5-pentamethylcyclohexanol **3** by using methylmagnesium iodide.

Also Jirgensons et al. (Eur. J. Med. Chem. 35 (2000) 555-565) disclose the manufacture of 1-hydroxy-1,3,3,5,5-pentamethylcyclohexane by reaction of 3,3,5,5-tetramethylcyclohexanone with a methylmagnesium halide. The product was purified by means of chromatography on a silica gel column.

In the third step of the sequence, said cyclohexanol 3 is converted into 1-chloroacetamido-1,3,3,5,5-pentamethylcyclohexane **6** by chloroacetonitrile in a Ritter reaction.

In the subsequent fourth step, cleavage of the chloroacetamido group in amide **6** with thiourea in acetic acid, and acidification of the resulting amine with hydrochloric acid in the fifth step of the reaction sequence results in 1-amino-1,3,3,5,5-pentamethylcyclohexane (Neramexane) **7** in the form of its hydrochloride.

WO 99/01416 discloses the preparation of alkylcyclohexanols employing alkylmagnesium iodide.

US 4,126,140 discloses the preparation of 1,3,5,5-tetramethytcyclohexa-1,3-diene by reacting isophorone with methylmagnesium chloride in tetrahydrofurane (Example I).

### OBJECTS OF THE INVENTION

One object of the invention is to improve one or more of the individual reaction steps of the above referenced reaction sequence in order to provide a method of preparing 1-amino-1,3,3,5,5-pentamethylcyclohexane or a pharmaceutically acceptable salt thereof that allows an advantageous realization on an economical industrial scale. It is in another object to minimize the amount of waste and/or unused chemicals produced during the manufacture of Neramexane or a pharmaceutically acceptable salt thereof. It is a further object to optimize or improve the yield and/or selectivity and/or product quality in regard to Neramexane or a pharmaceutically acceptable salt thereof. Particularly, the subject application aims to improve above step (ii), i.e. reaction of 3,3,5,5-pentamethylcyclohexanone to 1-hydroxy-1,3,3,5,5-pentamethylcyclohexane. Such an improved method may be regarded as one prerequisite for an advantageous manufacture of Neramexane or a pharmaceutically acceptable salt thereof on an economical industrial scale.

### SUMMARY OF THE INVENTION

The present invention relates to an improved synthesis of 1-hydroxy-1,3,3,5,5-pentamethylcyclohexane. Said compound is an intermediate in the manufacture of 1-amino-1,3,3,5,5-pentamethylcyclohexane (Neramexane) or a pharmaceutically acceptable salt thereof.

Specifically, the present invention relates to a method of preparing 1-hydroxy-1,3,3,5,5-pentamethylcyclohexane comprising step (ii):
(ii) converting 3,3,5,5-tetramethylcyclohexanone to 1-hydroxy-1,3,3,5,5-pentamethylcyclohexane in the presence of methylmagnesium chloride.

In one embodiment, in step (ii), a mixture comprising methylmagnesium chloride and an ether is reacted with 3,3,5,5-tetramethylcyclohexanone.

In another embodiment, in step (ii), 3,3,5,5-tetramethylcyclohexanone is added to a mixture comprising methylmagnesium chloride and an ether.

In one embodiment, the ether is tetrahydrofurane.

In one embodiment, a mixture comprising 3,3,5,5-tetramethylcyclohexanone and tetrahydrofurane is added to a mixture comprising methylmagnesium chloride and tetrahydrofurane.

In one embodiment, 1.2 to 1.75 molar equivalents methylmagnesium chloride are applied per molar equivalent 3,3,5,5-tetramethylcyclohexanone.

In one embodiment, the temperature is kept in a range of from 0 °C to 30 °C, or 15 °C to 25 °C.

The invention also relates to the use of methylmagnesium chloride for converting 3,3,5,5-tetramethylcyclohexanone to 1-hydroxy-1,3,3,5,5-pentamethylcyclohexane.

In one embodiment of the use, methylmagnesium chloride is dissolved in tetrahydrofurane.

According to another aspect, the invention also relates to a method of preparing 1-amino-1,3,3,5,5-pentamethylcyclohexane or a pharmaceutically acceptable salt thereof, such as the hydrochloride or the mesylate thereof, comprising step (ii):
(ii) converting 3,3,5,5-tetramethylcyclohexanone to 1-hydroxy-1,3,3,5,5-pentamethylcyclohexane in the presence of methylmagnesium chloride.

In one embodiment, said methylmagnesium chloride is free of ethylmagnesium chloride.

In one aspect, the invention relates to 1-amino-1,3,3,5,5-pentamethylcyclohexane or a pharmaceutically acceptable salt thereof which is substantially free of 1-amino-1-ethyl-3,3,5,5-tetramethylcylohexane and, optionally, free of 1-amino-3-ethyl-1,3,5,5-tetramethylcyclohexane; or a salt thereof.

It has unexpectedly been discovered that the method according to the invention results in a high yield in a product whose purity is sufficient to be employed in the subsequent step (iii) of the above referenced sequence without further purification. Thus, the method according to the invention simplifies the hitherto known method of producing Neramexane or a pharmaceutically acceptable salt thereof as referenced in the Background section of this application. It may be advantageously performed on an economical industrial scale.

### DETAILED DESCRIPTION OF THE INVENTION

This invention relates to a method of preparing 1-hydroxy-1,3,3,5,5-pentamethylcyclohexane from 3,3,5,5-tetramethylcyclohexanone.

Specifically, the invention relates to a method of preparing 1-hydroxy-1,3,3,5,5-pentamethylcyclohexane comprising step (ii):
(ii) converting 3,3,5,5-tetramethylcyclohexanone to 1-hydroxy-1,3,3,5,5-pentamethylcyclohexane in the presence of methylmagnesium chloride.

Methylmagnesium chloride is a Grignard reagent. It may be produced by reacting magnesium with methyl chloride.

The reaction according to step (ii) commonly is performed in a solvent.

In one embodiment, said solvent comprises an ether, or the solvent is an ether.

Ethers may be selected from diethyl ether, 1,4-dioxane, or tetrahydrofurane.

In one embodiment, said ether is tetrahydrofurane.

In one embodiment of the method of the invention, methylmagnesium chloride is added to 3,3,5,5-tetramethylcyclohexanone.

In another embodiment, 3,3,5,5-tetramethylcyclohexanone is added to methylmagnesium chloride.

In one embodiment, a solution of methylmagnesium chloride in tetrahydrofurane is added to a solution of 3,3,5,5-tetramethylcyclohexanone in tetrahydrofurane.

In another embodiment, a solution of 3,3,5,5-tetramethylcyclohexanone in tetrahydrofurane is added to a solution of methylmagnesium chloride in tetrahydrofurane.

In one embodiment, the concentration of methylmagnesium chloride in tetrahydrofurane is from 15 to 30 % by weight, or 20 to 25 % by weight based on the total amount of methylmagnesium chloride and tetrahydrofurane.

In one embodiment, the concentration of methylmagnesium chloride in tetrahydrofurane is 23 % by weight based on the total amount of methylmagnesium chloride and tetrahydrofurane.

Accordingly, in one embodiment, a mixture comprising methylmagnesium chloride and tetrahydrofurane is reacted with a mixture comprising 3,3,5,5-tetramethylcyclohexanone and tetrahydrofurane.

In one embodiment, more than one molar equivalent methylmagnesium chloride is employed per one molar equivalent 3,3,5,5-tetramethylcyclohexanone, e.g. from 1.1 to 2.0 molar equivalents.

In another embodiment, about 1.2 to 1.75 molar equivalents methylmagnesium chloride are employed per molar equivalent 3,3,5,5-tetramethylcyclohexanone.

In one embodiment, a solution of 3,3,5,5-tetramethylcyclohexanone in tetrahydrofurane is added to a solution of methylmagnesium chloride in tetrahydrofurane, which contains 1.2 to 1.75 molar equivalents methylmagnesium chloride per molar equivalent 3,3,5,5-tetramethylcyclohexanone.

In one embodiment, the conversion is performed such that the temperature is controlled.

In one embodiment, the conversion is performed such that the temperature may be maintained in a relatively narrow temperature range.

In one embodiment, the conversion in step (ii) is performed at a temperature of from -5 °C to 30 °C, or 0 °C to 30 °C, or 0 °C to 25 °C, or 0 °C to 20 °C, or 5 to 20 °C, or 10°C to 25 °C, or 15 to 25 °C.

The Grignard reagent may be added to 3,3,5,5-tetramethylcyclohexanone rather fast, provided that the selected temperature window may be maintained, and the subsequent reaction commonly proceeds rather fast. Usually, the reaction may be terminated after three hours or two hours or even one hour, depending on the reaction temperature employed.

After the completion of the reaction, the reaction mixture may be treated with water in order to destroy an excess of Grignard reagent, respectively to destroy basic magnesium compounds.

In one embodiment, an acid such as hydrochloric acid, or an ammonium salt, is added to support the formation of 1-hydroxy-1,3,3,5,5-pentamethylcyclohexane.

In one embodiment, the formed organic layer is separated off from the aqueous layer. Subsequently, the organic layer may be concentrated by removing volatile organic compounds in vacuo. The residue is crude 1-hydroxy-1,3,3,5,5-pentamethylcyclohexane.

In one embodiment, the 1-hydroxy-1;3,3,5,5-pentamethylcyclohexane formed in step (ii) is obtained by extracting the aqueous mixture with an appropriate organic solvent such as methylene chloride or toluene or petroleum ether. Subsequent to extracting, the solvent is removed by distillation. The liquid residue comprising crude 1-hydroxy-1,3,3,5,5-pentamethylcyclohexane as obtained may be employed without purification in step (iii) of the reaction sequence.

In another embodiment, subsequent to extracting, the extract may be dried according to known methods. For example, the extract may be dried over sodium sulphate. After separating off said sulphate by filtration, the solvent may be removed by distillation. The liquid residue comprising crude 1-hydroxy-1,3,3,5,5-pentamethylcyclohexane as obtained may be employed without purification in step (iii) of the reaction sequence.

Commonly, the yield of target compound 1-hydroxy-1,3,3,5,5-pentamethylcyclohexane in step (ii) is high.

In one embodiment, the yield of crude 1-hydroxy-1,3,3,5,5-pentamethylcyclohexane ranges between 90 % and 100 % by weight.

In one embodiment, the crude product contains the target compound 1-hydroxy-1,3,3,5,5-pentamethylcyclohexane in an amount of at least 94 % by weight as determined by gas-liquid chromatography.

In one embodiment, the obtained crude 1-hydroxy-1,3,3,5,5-pentamethylcyclohexane may be further purified e.g. by distillation or chromatography.

However, due to the high purity of the crude target compound resulting from the use of methylmagnesium chloride as the Grignard reagent, said target compound may be employed in the next step of the reaction sequence, the third step as discussed in the Background section, as the crude product, i.e. as a product that is neither purified by distillation nor chromatography, or that is not purified at all.

Accordingly, the use of methylmagnesium chloride for converting 3,3,5,5-tetramethylcyclohexane to 1-hydroxy-1,3,3,5,5-pentamethylcyclohexane is advantageous over the respective uses of methylmagnesium bromide and methylmagnesium iodide. This particularly concerns the achievable high yield and the possibility to apply the obtained compound as crude product in the reaction sequence as addressed in the Background section. This is particularly advantageous in view of an industrial realization.

Accordingly, the present invention also relates to the use of methylmagnesium chloride for converting 3,3,5,5-tetramethylcyclohexanone to 1-hydroxy-1,3,3,5,5-pentamethylcyclohexane.

In one embodiment of the use, methylmagnesium chloride is dissolved in tetrahydrofurane.

1-hydroxy-1,3,3,5,5-pentamethylcyclohexane prepared according to the method of the invention may be used for preparing 1-amino-1,3,3,5,5-pentamethylcyclohexane (Neramexane) or a pharmaceutically acceptable salt thereof.

Accordingly, in another aspect, the invention also relates to a method of preparing 1-amino-1,3,3,5,5-pentamethylcyclohexane or a pharmaceutically acceptable salt thereof, comprising step (ii):
(ii) converting 3,3,5,5-tetramethylcyclohexanone to 1-hydroxy-1,3,3,5,5-pentamethylcyclohexane in the presence of methylmagnesium chloride.

For the purpose of this disclosure, the term *"pharmaceutically acceptable salts"* refers to salts of neramexane that are physiologically tolerable and do not typically produce untoward reactions when administered to a mammal (e.g., human). Typically, the term *"pharmaceutically acceptable salt"* means approved by a regulatory agency of the Federal or a state government or listed in the U.S. Pharmacopeia or other generally recognized pharmacopeia for use in mammals, and more particularly in humans.

Conversion of 1-amino-1,3,3,5,5-pentamethylcyclohexane to a pharmaceutically acceptable salt thereof is accomplished in conventional fashion by admixture of the base with at least one molecular equivalent of a selected acid in an inert organic solvent. Isolation of the salt is carried out by techniques known to the art such as inducing precipitation with a non-polar solvent (e.g. ether) in which the salt has limited solubility. The nature of the salt is not critical, provided that it is non-toxic and does not substantially interfere with the desired pharmacological activity.

Examples of pharmaceutically acceptable salts are those formed with hydrochloric, hydrobromic, methanesulfonic, acetic, succinic, maleic, citric acid and related acids.

Further pharmaceutically acceptable salts include, but are not limited to, acid addition salts, such as those made with hydroiodic, perchloric, sulfuric, nitric, phosphoric, propionic, glycolic, lactic, pyruvic, malonic, fumaric, tartaric, benzoic, carbonic, cinnamic, mandelic, ethanesulfonic, hydroxyethanesulfonic, benezenesulfonic, p-toluene sulfonic, cyclohexanesulfamic, salicyclic, p-aminosalicylic, 2-phenoxybenzoic, and 2-acetoxybenzoic acid.

In one embodiment, step (ii) is effected as defined in any one of the above embodiments.

### 1-hydroxy-1-ethyl-3,3,5,5-tetramethylcyclohexane as a possible by-product

In one embodiment, 1-hydroxy-1-ethyl-3,3,5,5-tetramethylcyclohexane may be formed as a by-product in step (ii). This product may e.g. be detected by gas chromatographical analysis.

In one embodiment, the occurrence of 1-hydroxy-1-ethyl-3,3,5,5-tetramethylcyclohexane may be attributed to the addition of an ethyl group instead of a methyl group to the carbonyl group of 3,3,5,5-tetramethylcyclohexanone in step (ii).

In one embodiment, the occurrence of said by-product may be attributed to the contamination of the employed methylmagnesium Grignard reagent with an ethylmagnesium Grignard reagent such as ethylmagnesium chloride.

In one embodiment, the occurrence of said by-product may be suppressed or prevented by employing a purified methylmagnesium Grignard reagent which is free of an ethylmagnesium Grignard reagent such as ethylmagnesium chloride.

In one embodiment, methylmagnesium chloride contains less than 1 % by weight ethylmagnesium chloride based on the total amount of methylmagnesium chloride and ethylmagnesium chloride, or less than 0.5 % by weight, or less than 0.1 % by weight.

In one embodiment, it is not necessary to remove said by-product from the crude 1-hydroxy-1,3,3,5,5-pentamethylcyclohexane as obtained in step (ii).

Subsequent to a possible formation in step (ii), 1-hydroxy-1-ethyl-3,3,5,5-tetramethylcyclohexane reacts in a similar manner as 1-hydroxy-1,3,3,5,5-pentamethylcyclohexane as referenced in the reaction sequence of the Background section. Reaction with chloroacetonitrile results in the corresponding Ritter product which subsequently is converted with thiourea to the corresponding amine, respectively after acidification to a salt thereof.

Thus, if starting from the product obtained in step (ii), the subsequent synthesis of neramexane or a salt thereof is performed, 1-amino-1-ethyl-3,3,5,5-tetramethylcyclohexane respectively a salt thereof may be formed as a by-product.

In one embodiment, said by-product may be removed from the target product neramexane by purifying 1-amino-1,3,3,5,5-pentamethylcyclohexane prior to the salt formation. In one embodiment, the amine may be purified by distillation wherein the by-product is removed.

In another embodiment, the neramexane salt obtained after acidification is purified. In one embodiment, said salt may be purified by a step of re-crystallization employing a suitable solvent.

In one embodiment, the salt of 1-amino-1,3,3,5,5-pentamethylcyclohexylamine is the mesylate and the salt which is removed by recrystallization is 1-amino-1-ethyl-3,3,5,5-tetramethylcyclohexane mesylate. In one embodiment, the solvent used for re-crstallization is anisole.

In another embodiment, it is conceivable that 3,3,5,5-tetramethylcylohexanone as used as starting material in step (ii) is also contaminated with an ethyl compound, provided that said starting material is prepared from isophorone using a Grignard reagent such as methylmagnesium chloride. The occurrence of 3-ethyl-3,5,5-trimethylcyclohexanone in 3,3,5,5-tetramethylcylohexanone may be attributed to the addition of an ethyl group instead of a methyl group to isophorone to yield the respective cyclohexanone. This cyclohexanone reacts in a similar manner as 3,3,5,5-pentamethylcyclohexanone in the subsequent reactions. Reaction with methylmagesium chloride results in 1-hydroxy-3-ethyl-1,3,5,5-tetramethylcyclohexane. Reaction with chloroacetonitrile results in the corresponding Ritter product which subsequently is converted with thiourea to the corresponding amine (1-amino-3-ethyl-1,3,5,5-tetramethylcyclohexane), respectively after acidification to a salt thereof.

Since 1-amino-3-ethyl-1,3,5,5-tetramethylcyclohexane has two chiral centers, two diastereomers are formed. The formation of said compounds may be suppressed or prevented, respectively the removal of said compounds may be performed by the methods as described above in connection with 1-hydroxy-1-ethyl-3,3,5,5-tetramethylcyclohexane.

The yield of crude 1-hydroxy-1,3,3,5,5-pentamethylcyclohexane in the process according to the invention is approx. 100 %. The purity of the crude product is sufficient to be employed in the subsequent step (iii) of the referenced sequence without further purification. Thus, the method according to the invention simplifies the hitherto known method of producing Neramexane or a pharmaceutically acceptable salt thereof as referenced in the Background section of this application. It may be advantageously performed on an economical industrial scale.

### EXAMPLE

A mixture of 153 g 3,3,5,5-tetramethylcyclohexanone in 153 g tetrahydrofurane is added by dropping to a stirred mixture of 93 g methylmagnesium chloride and 372 g tetrahydrofurane. The dropping rate is selected such that the temperature of the mixture is kept between 5 and 15 °C. After the addition is terminated, the mixture is stirred for another 60 minutes. Subsequently, diluted hydrochloric acid is added to decompose an excess of methylmagnesium chloride, and to decompose basic magnesium compounds. The mixture is extracted twice with petroleum ether. The extracts are combined and the solvent is distilled off. The yield of crude 1-hydroxy-1,3,3,5,5-pentamethylcyclohexane is quantitative (170 g). The content of target compound in the crude product is about 95 % by weight as determined by gas-liquid chromatography.

## Claims

1. Method of preparing 1-hydroxy-1,3,3,5,5-pentamethylcyclohexane comprising step (ii):
(ii) converting 3,3,5,5-tetramethylcyclohexanone to 1-hydroxy-1,3,3,5,5-pentamethylcyclohexane in the presence of methylmagnesium chloride.

2. Method according to claim 1, wherein in step (ii) a mixture comprising methylmagnesium chloride and an ether is reacted with 3,3,5,5-tetramethylcyclohexanone.

3. Method according to claim 1 or 2, wherein 3,3,5,5-tetramethylcyclohexanone is added to a mixture comprising methylmagnesium chloride and an ether.

4. Method of claim 2 or 3, wherein the ether is tetrahydrofurane.

5. Method of any one of the preceeding claims, wherein a mixture comprising 3,3,5,5-tetramethylcyclohexanone and tetrahydrofurane is added to a mixture comprising methylmagnesium chloride and tetrahydrofurane.

6. Method according to any one of the preceding claims, wherein 1.2 to 1.75 molar equivalents methylmagnesium chloride are applied per one molar equivalent 3,3,5,5-tetramethylcyclohexanone.

7. Method according to any one of the preceding claims, wherein the temperature is kept in a range of from 0 °C to 30 °C.

8. Method according to any one of the preceding claims, wherein the temperature is kept in a range of from 15 °C to 25 °C.

9. Use of methylmagnesium chloride for converting 3,3,5,5-tetramethylcyclohexanone to 1-hydroxy-1,3,3,5,5-pentamethylcyclohexane.

10. Use according to claim 9, wherein methylmagnesium chloride is dissolved in tetrahydrofurane.

11. Method of preparing 1-amino-1,3,3,5,5-pentamethylcyclohexane or a pharma-ceutically acceptable salt thereof, comprising carrying out step (ii) according to claim 1.

12. Method according to claim 11, wherein step (ii) is effected according to any one of claims 2 to 8.

13. Method according to any one claims 1 to 8 or 11 to 12, wherein said methylmagnesium chloride is free of ethylmagnesium chloride.

14. Use according to claim 9 or 10, wherein said methylmagnesium chloride is free of ethylmagnesium chloride.

15. Method according to claim 11 or 12, wherein said pharmaceutically acceptable salt is the hydrochloride or the mesylate.

## Patentansprüche

1. Verfahren zur Herstellung von 1-Hydroxy-1,3,3,5,5-pentamethylcyclohexan umfassend die Stufe (ii):
(ii) Überführen von 3,3,5,5-Tetramethylcyclohexanon in 1-Hydroxy-1,3,3,5,5-pentamethylcyclohexan in Gegenwart von Methylmagnesiumchlorid.

2. Verfahren nach Anspruch 1, wobei in Stufe (ii) eine Mischung umfassend Methylmagnesiumchlorid und ein Ether mit 3,3,5,5-Tetramethylcyclohexanon reagiert wird.

3. Verfahren nach Anspruch 1 oder 2, wobei 3,3,5,5-Tetramethylcyclohexanon einer Mischung umfassend Methylmagnesiumchlorid und einen Ether hinzugefügt wird.

4. Verfahren nach Anspruch 2 oder 3, wobei der Ether Tetrahydrofuran ist.

5. Verfahren nach einem der vorstehenden Ansprüche, wobei eine Mischung umfassend 3,3,5,5-Tetramethylcyclohexanon und Tetrahydrofuran einer Mischung umfassend Methylmagnesiumchlorid und Tetrahydrofuran zugesetzt wird.

6. Verfahren nach einem der vorstehenden Ansprüche, wobei 1,2 bis 1,75 Molequivalente Methylmagnesiumchlorid pro einem Molequivalent 3,3,5,5-Tetramethylcyclohexanon angewendet werden.

7. Verfahren nach einem der vorstehenden Ansprüche, wobei die Temperatur in einem Bereich von 0 °C bis 30 °C gehalten wird.

8. Verfahren nach einem der vorstehenden Ansprüche, wobei die Temperatur in einem Bereich von 15 °C bis 25 °C gehalten wird.

9. Verwendung von Methylmagnesiumchlorid zum Überführen von 3,3,5,5-Tetramethylcyclohexanon in 1-Hydroxy-1,3,3,5,5-pentamethylcyclohexan.

10. Verwendung nach Anspruch 9, wobei Methylmagnesiumchlorid in Tetrahydrofuran gelöst wird.

11. Verfahren zur Herstellung von 1-Amino-1,3,3,5,5-pentamethylcyclohexan oder einem pharmazeutisch verträglichen Salz davon, umfassend das Ausführen von Stufe (ii) nach Anspruch 1.

12. Verfahren nach Anspruch 11, wobei Stufe (ii) nach einem der Ansprüche 2 bis 8 ausgeführt wird.

13. Verfahren nach einem der Ansprüche 1 bis 8 oder 11 bis 12, wobei besagtes Methylmagnesiumchlorid frei von Ethylmagnesiumchlorid ist.

14. Verwendung nach Anspruch 9 oder 10, wobei besagtes Methylmagnesiumchlorid frei von Ethylmagnesiumchlorid ist.

15. Verfahren nach Anspruch 11 oder 12, wobei besagtes pharmazeutisch verträgliches Salz das Hydrochlorid oder das Mesylat ist.

## Revendications

1. Procédé de préparation du 1-hydroxy-1,3,3,5,5-pentaméthylcyclohexane comprenant l'étape (ii) :
(ii) conversion de la 3,3,5,5-tétraméthylcyclohexanone en 1-hydroxy-1,3,3,5,5-pentaméthylcyclohexane en présence de chlorure de méthylmagnésium.

2. Procédé selon la revendication 1 où dans l'étape (ii) un mélange comprenant du chlorure de méthylmagnésium et un éther est mis à réagir avec la 3,3,5,5-tétraméthylcyclohexanone.

3. Procédé selon la revendication 1 ou 2 où la 3,3,5,5-tétraméthylcyclohexanone est ajoutée à un mélange comprenant du chlorure de méthylmagnésium et un éther.

4. Procédé selon la revendication 2 ou 3 où l'éther est le tétrahydrofurane.

5. Procédé selon l'une quelconque des revendications précédentes où un mélange comprenant de la 3,3,5,5-tétraméthylcyclohexanone et du tétrahydrofurane est ajouté à un mélange comprenant du chlorure de méthylmagnésium et du tétrahydrofurane.

6. Procédé selon l'une quelconque des revendications précédentes où 1,2 à 1,75 équivalent molaire de chlorure de méthylmagnésium est appliqué par équivalent molaire de 3,3,5,5-tétraméthylcyclohexanone.

7. Procédé selon l'une quelconque des revendications précédentes où la température est maintenue dans une plage de 0°C à 30°C.

8. Procédé selon l'une quelconque des revendications précédentes où la température est maintenue dans une plage de 15°C à 25°C.

9. Utilisation du chlorure de méthylmagnésium pour convertir la 3,3,5,5-tétraméthylcyclohexanone en 1-hydroxy-1,3,3,5,5-pentaméthyl-cyclohexane.

10. Utilisation selon la revendication 9 où le chlorure de méthylmagnésium est dissous dans le tétrahydrofurane.

11. Procédé de préparation du 1-amino-1,3,3,5,5-pentaméthylcyclohexane ou d'un sel pharmaceutiquement acceptable de celui-ci comprenant la mise en oeuvre de l'étape (ii) selon la revendication 1.

12. Procédé selon la revendication 11 où l'étape (ii) est accomplie selon l'une quelconque des revendications 2 à 8.

13. Procédé selon l'une quelconque des revendications 1 à 8 ou 11à 12 où ledit chlorure de méthylmagnésium est dépourvu de chlorure d'éthylmagnésium.

14. Utilisation selon la revendication 9 ou 10 où ledit chlorure de méthylmagnésium est dépourvu de chlorure d'éthylmagnésium.

15. Procédé selon la revendication 11 ou 12 où ledit sel pharmaceutiquement acceptable est le chlorhydrate ou le mésylate.
